(19) Europäisches Patentamt
European Patent Office
Office européen des brevets

(11) Veröffentlichungsnummer: **0 124 948**
**B1**

(12) # EUROPÄISCHE PATENTSCHRIFT

(45) Veröffentlichungstag der Patentschrift: **07.01.88**

(51) Int. Cl.⁴: **H 01 C 17/06,** H 01 C 7/06

(21) Anmeldenummer: **84200642.1**

(22) Anmeldetag: **07.05.84**

(54) **Widerstandspaste für einen elektrischen Widerstand.**

(30) Priorität: **09.05.83 NL 8301631**

(43) Veröffentlichungstag der Anmeldung:
**14.11.84 Patentblatt 84/46**

(45) Bekanntmachung des Hinweises auf die Patenterteilung:
**07.01.88 Patentblatt 88/1**

(84) Benannte Vertragsstaaten:
**BE CH DE FR GB IT LI NL**

(56) Entgegenhaltungen:
**US - A - 3 681 262**
**US - A - 4 107 387**

(73) Patentinhaber: **N.V. Philips' Gloeilampenfabrieken, Groenewoudseweg 1, NL-5621 BA Eindhoven (NL)**

(72) Erfinder: **Boonstra, Alexander Hendrik, c/o INT. OCTROOIBUREAU B.V. Prof. Holstlaan 6, NL-5656 AA Eindhoven (NL)**
Erfinder: **Mutsaers, Cornelis Adrianus H. A., c/o INT. OCTROOIBUREAU B.V. Prof. Holstlaan 6, NL-5656 AA Eindhoven (NL)**
Erfinder: **van der Kruijs, Franciscus Nicolaas G. R., c/o INT. OCTROOIBUREAU B.V. Prof. Holstlaan 6, NL-5656 AA Eindhoven (NL)**

(74) Vertreter: **Auwerda, Cornelis Petrus et al, INTERNATIONAAL OCTROOIBUREAU B.V. Prof. Holstlaan 6, NL-5656 AA Eindhoven (NL)**

**EP 0 124 948 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung bezieht sich auf eine Widerstandspaste für einen elektrischen Widerstand, bestehend aus einem Gemisch aus einer metalloxidischen Verbindung, einem ständig in der Paste verbleibenden und einem vorübergehend in der Paste vorhandenen Bindemittel und auf einen elektrischen Widerstand, bestehend aus auf einem Substrat angebrachten, mit Anschlussdrähten versehenen Widerstandsmaterial aus der, durch Erhitzung von dem vorübergehend in der Paste vorhandenen Bindemittel befreiten, Widerstandspaste.

Aus der US Patentschrift 3 681 262 ist eine Widerstandspaste bekannt, um daraus Widerstandskörper herzustellen. Die in der Paste verwendeten metalloxidischen Verbindungen müssen der nachfolgenden allgemeinen Formel entsprechen:

$(M_xBi_{2-x})(M'_yM''_{2-y})O_{7-z}$,

worin M mindestens ein Metall aus der Gruppe Y, Tl, In, Cd, Pb und einem seltenen Erdmetall mit der Atomnummer von 57 bis 71 ist, M′ mindestens ein Metall aus der Gruppe Pt, Ti, Sn, Cr, Rh, Re, Zr, Sb und Ge ist,
M″ mindestens eines der Metalle Ru und Ir ist,
x eine Zahl zwischen 0 und 2 ist,
y eine Zahl zwischen 0 und 2 ist und
z eine Zahl von 0 bis 1 ist, die wenigstens etwa gleich x/2 ist, falls
M ein zweiwertiges Metall ist. Unter diese Klasse von metalloxidischen Verbindungen fällt auch eine Verbindung mit der Formel

$Pb_2Rh_yRu_{2-y}O_{7-z}$.

Ein Nachteil der meisten der Zusammensetzungen ist, dass der Temperaturkoeffizient des Widerstandes in dem für den praktischen Gebrauch interessanten Temperaturbereich von -55 bis +150°C ziemlich gross ist und innerhalb dieses Bereiches ziemlich stark temperaturabhängig verläuft. Ein weiterer Nachteil, der sehr oft auftritt, ist, dass der Widerstandswert sich im Laufe der Zeit stark ändert.

Die Erfindung hat nun zur Aufgabe, eine derartige Paste und einen damit hergestellten Widerstandskörper zu schaffen, der einen Widerstandswert hat, der während der ganzen Lebensdauer des Widerstandskörpers innerhalb einer Toleranzgrenze von nicht mehr als ±5 ‰ verläuft. Ausserdem muss der Temperaturkoeffizient des Widerstands innerhalb des Temperaturbereiches von -55 bis +150°C zwischen $-10\times10^{-6}$/°C und $+10\times10^{-6}$/°C bleiben. Unter Lebensdauer wird in diesem Zusammenhang ein Zeitraum von 25 Jahren bei einer Temperatur von 50°C verstanden. Als Massstab wird dafür eine beschleunigte Dauerprobe während 200 Stunden bei 200°C durchgeführt.

Diese Aufgabe wird dadurch gelöst, dass die metalloxidische Verbindung die untenstehende Formel aufweist

$Pb_2Rh_yRu_{2-y}O_{7-z}$
worin $0,15 \leqq y \leqq 0.95$ und $O \leqq z \leqq 0,5$ ist.

Das Erhalten einer derart hohen Stabilität des nach dem Brennen erhaltenen Widerstandskörpers während der Gebrauchsdauer desselben ist der Tatsache zuzuschreiben, dass die Bestandteile der widerstandsbestimmenden metalloxidischen Verbindung eine zu vernachlässigende Migration in den üblichen, als ständig in der Paste verbleibendem Bindemittel (Dauerbindemittel) verwendeten Gläsern aufweist.

Ein in dieser Hinsicht als bevorzugtes Zusammensetzungsgebiet zu bezeichnender Glastyp ist gekennzeichnet durch die Bestandteile PbO, $SiO_2$, $BO_{1,5}$ und gegebenenfalls $AlO_{1,5}$ zwischen den Grenzen in Mol%:

| | |
|---|---|
| $SiO_2$ | 10–45 |
| PbO | 25–35 |
| $BO_{1,5}$ | 20–60 |
| $AlO_{1,5}$ | 0–15. |

Beim Einstellen des Pegels des spezifischen Widerstandes verfügt man über die Variationsmöglichkeit des Verhältnisses der widerstandsbestimmenden metalloxidischen Verbindung zu dem Dauerbindemittel, wobei der widerstandsbestimmende Bestandteil mehr oder weniger «verdünnt» wird. Der Temperaturkoeffizient des Widerstandes wird durch eine richtige Wahl von y äusserst genau auf den gewünschten Wert eingestellt. Infolge der zu vernachlässigenden Migration der Bestandteile der metalloxidischen Verbindung wird in der Lebensdauer ein sehr konstanter Temperaturkoeffizient des Widerstandes gemessen.

Eine Variationsmöglichkeit zur Einstellung des Wertes des spezifischen Widerstandes ist die Hinzufügung eines isolierenden Oxids, wie Aluminiumoxid oder Titaniumdioxid, das sich nur schwer in den übrigen Bestandteilen löst bzw. mit denselben reagiert.

Zur Erläuterung der Erfindung folgen untenstehend einige Ausführungsbeispiele. Die in den Beispielen verwendeten Verbindungen $Pb_2Rh_yRu_{2-y}O_{7-z}$ werden wie folgt dargestellt. Es wird ausgegangen von einer basischen Lösung von Kaliumruthenat. Dieser Lösung wird die durch den gewünschten Wert von y bestimmte Menge $Rh(NO_3)_3$-Lösung hinzugefügt. Der End-pH-Wert der Lösung muss grösser bleiben als 12. Dem Gemisch wird eine dreifache Menge (Mol) $Pb(NO_3)_2$-Lösung gegenüber der Summe von Ruthenat und Rhodat zugefügt. Auch dabei muss für einen End-pH-Wert grösser als 8 gesorgt werden, gegebenenfalls durch Hinzufügung von KOH. Der entstandene Niederschlag wird abfiltriert, zweimal mit Wasser gewaschen und daraufhin nach Trocknen während 1 Stunde an der Luft bei 700°C gebrannt. Danach wird der Überschuss an PbO mit verdünnter $HNO_3$ entfernt, und der Rest wird bei 150°C in Luft getrocknet.

Beispiel 1.

Glaspulver mit einer mittleren Teilchengrösse von etwa 1 µm und einer Zusammensetzung:

| | | |
|---|---|---|
| PbO | 26,1 Mol% | 61 Gew.% |
| $SiO_2$ | 38,2 Mol% | 24 Gew.% |
| $BO_{1,5}$ | 24,5 Mol% | 9 Gew.% |
| $AlO_{1,5}$ | 11,2 Mol% | 6 Gew.% |

wird in einem Gewichtsverhältnis 1:1 mit dem Widerstandsmaterial $Pb_2Rh_{0,80}Ru_{1,20}O_{6,5}$ mit einer Teilchengrösse kleiner als 0,2 µm gemischt. Daraufhin wird das Pulver mit einem oder mehreren Bindemitteln und Lösungsmitteln, beispielsweise Äthylcellulose in Benzylbenzoat, zu einer Paste verarbeitet. Die Paste wird durch Siebdrucken auf einem aus Aluminiumoxid bestehenden Substrat, das vorher mit AgPd-Kontakten versehen ist, angebracht. Die bedruckten Substrate werden getrocknet und daraufhin entsprechend dem Brennprogramm mit 850°C als Spitzentemperatur erhitzt.

Bei einer Schichtdicke von 15 µm haben die Flächenwiderstände einen Wert von 2,5 kΩ, einen $TC_R$ von $-8\times10^{-6}$/°C im Temperaturbereich von -55 bis +20°C und einen $TC_R$ von $+7\times10^{-6}$/°C im Temperaturbereich von +20 bis +150°C.

Beispiel 2.

Es wird eine Siebdruckpaste hergestellt mit dem Glaspulver aus Beispiel 1 und mit dem Widerstandsmaterial $Pb_2Rh_{0,25}Ru_{1,75}O_{6,5}$, das eine Teilchengrösse unter 0,2 µm hat in einem Gewichtsverhältnis 6:1. Die im Siebdruckverfahren hergestellten Widerstandsmuster auf einem Substrat, wie in dem Beispiel 1, werden nach dem Trocknen einem Brennprogramm ausgesetzt mit einer Spitzentemperatur von 750°C. Die erhaltenen Flächenwiderstände haben einen Wert von 0,3 MΩ bei einer Schichtdicke von 15 µm, einen $TC_R$ im Temperaturbereich von -55 bis +20°C von $-6\times10^{-6}$/°C und in dem Bereich von +20 bis +150°C von $+9\times10^{-6}$/°C.

Beispiel 3.

Glaspulver mit einer mittleren Teilchengrösse von etwa 1 µm und der folgenden Zusammenstellung:

| | | |
|---|---|---|
| PbO | 29,5 Mol% | 65 Gew.% |
| $SiO_2$ | 43,0 Mol% | 25,5 Gew.% |
| $BO_{1,5}$ | 27,5 Mol% | 9,5 Gew.% |

wird in einem Gewichtsverhältnis von 1:4 mit dem Widerstandsmaterial $Pb_2Rh_{0,5}Ru_{1,5}O_{6,5}$ gemischt und zu einer Paste verarbeitet. Davon werden auf einem $Al_2O_3$-Substrat Widerstände im Siebdruckverfahren hergestellt und das Ganze wird einem Brennprogramm mit einer Spitzentemperatur von 850°C ausgesetzt. Die erhaltenen Widerstände haben bei einer Schichtdicke von 15 µm einen Wert für den Flächenwiderstand von etwa 50 kΩ. Der $TC_R$ beträgt:

$-9\times10^{-6}$/°C (-55→+20°C) und
$+6\times10^{-6}$/°C (+20→+150°C).

Beispiel 4.

Der in Beispiel 3 beschriebenen Paste werden 2 Gew.% $TiO_2$ hinzugefügt. Das Substrat mit den darauf im Siebdruckverfahren angebrachten Widerständen wird einem Brennprozess mit einer Spitzentemperatur von 750°C ausgesetzt. Die Widerstände zeigen einen Flächenwiderstandswert von 0,8 MΩ bei einer Schichtdicke von 15 µm. Der $TC_R$ beträgt:

$-9\times10^{-6}$/°C (-55→+20°C)
$+5\times10^{-6}$/°C (+20→+150°C).

Die Widerstände werden zur Erprobung ihrer Stabilität einer beschleunigten Dauerprobe während 200 Stunden bei 200°C in Luft ausgesetzt.

Die Widerstandswerte aller obenstehend beschriebenen Widerstände zeigten am Ende der Probe Abweichungen von weniger als ±5‰.

**Patentansprüche**

1. Widerstandspaste für einen elektrischen Widerstandskörper, dessen Widerstandswert sich während der ganzen Lebensdauer des Widerstandskörpers um nicht mehr als ±5 Promille verändert und der einen Temperaturkoeffizienten des Widerstands zwischen $-10\times10^{-6}$°C und $+10\times10^{-6}$°C im Temperaturbereich von -55 bis +150°C besitzt, bestehend aus einem Gemisch aus einer metalloxidischen Verbindung, einem permanenten Bindemittel und einem vorübergehend in der Paste vorhandenen Bindemittel, wobei die metalloxidische Verbindung die nachfolgende Formel aufweist:

$Pb_2Rh_yRu_{2-y}O_{7-z}$,

worin $0.15 \leqq y \leqq 0.95$ und $O \leqq z \leqq 1/2$ ist.

2. Widerstandspaste nach Anspruch 1, dadurch gekennzeichnet, dass das ständig in der Paste verbleibende Bindemittel ein Glas ist mit der folgenden Zusammensetzung (Angaben in Mol%):

| | |
|---|---|
| $SiO_2$ | 10-45 |
| PbO | 25-35 |
| $BO_{1,5}$ | 20-60 |
| $AlO_{1,5}$ | 0-15. |

3. Widerstandspaste nach Anspruch 1 oder 2, dadurch gekennzeichnet, dass diese zugleich ein isolierendes Oxid enthält, das in den übrigen Bestandteilen schwer löslich ist bzw. mit denselben schwer reagiert.

4. Verwendung der Widerstandspaste nach den Ansprüchen 1 bis 3 für die Herstellung von elektrischen Widerständen.

## Claims

1. A resistor paste for an electric resistor body whose resistance value during the whole life of the resistor body does not vary by more than ±5‰ and which has a temperature coefficient of the resistance between $-10\times10^{-6}/°C$ and $+10\times10^{-6}/°C$ in the temperature range from −55 to +150°C, consisting of a mixture of a metal oxidic compound, a permanent binder and a temporary binder in the paste, in which the metal oxidic compound has the following formula:

$Pb_2Rh_yRu_{2-y}O_{7-z}$
wherein $0.15 \leqq y \leqq 0.95$ and $O \leqq z \leqq 1/2$.

2. A resistor paste as claimed in Claim 1, characterized in that the permanent binder in the paste is a glass having a composition (in mol.%):

| | |
|---|---|
| $SiO_2$ | 10–45 |
| PbO | 25–35 |
| $BO_{1.5}$ | 20–60 |
| $AlO_{1.5}$ | 0–15. |

3. A resistor paste as claimed in Claim 1 or 2, characterized in that it also comprises an insulating oxide which is poorly soluble in and poorly reacts with, respectively, the remaining components.

4. The use of the resistor paste as claimed in Claim 1 to 3 for the manufacture of electric resistors.

## Revendications

1. Pâte résistante pour un corps de résistance, dont la valeur ohmique ne varie pas plus qu'environ ±5 pourmille pendant toute la durée de vie du corps de résistance et qui présente un coefficient de température de la résistance entre $-10\times10^{-6}/°C$ et $+10\times10^{-6}/°C$ dans la gamme de températures comprise entre −55 et +150°C, constituée par un mélange d'un composé d'oxyde métallique d'un liant permanent et d'un liant présent de façon temporaire dans la pâte, le composé d'oxyde métallique présentant la formule suivante:

$Pb_2Rh_yRu_{2-y}O_{7-z}$
formule dans laquelle $0.15 \leqq y \leqq 0.95$ et $O \leqq z \leqq 1/2$.

2. Pâte de résistance selon la revendication 1, caractérisée en ce que le liant présent de façon permanente dans la pâte est un verre présentant la composition en % en moles suivantes:

| | |
|---|---|
| $SiO_2$ | 10–45 |
| PbO | 25–35 |
| $BO_{1,5}$ | 20–60 |
| $AlO_{1,5}$ | 0–15 |

3. Pâte de résistance selon la revendication 1 ou 2, caractérisé en ce que celle-ci contient simultanément un oxyde isolant, qui ne se dissout guère dans les autres composants ou ne réagit guère avec ces derniers.

4. Utilisation de la pâte de résistance selon les revendications 1 à 3, pour la réalisation de résistances électriques.